# EUROPEAN PATENT APPLICATION

(11) **EP 1 069 433 A2**
(43) Date of publication of application: **17.01.2001**
(21) Application number: 00114143.1
(22) Date of filing: 11.07.2000
(51) Int. Cl.: G01N 33/543, G01N 33/553, G01N 33/52, G01N 33/58

(54) **Agglutination assay method in porous medium layer**

(30) Priority: 12.07.1999 JP 19741999
(71) Applicant: FUJI PHOTO FILM CO., LTD., Kanagawa 250-01 (JP)
(72) Inventor: Nakamura, Kentaro, Asaka-shi, Saitama (JP); Kawasaki, Kazuya, Asaka-shi, Saitama (JP); Seshimoto, Osamu, Asaka-shi, Saitama (JP); Nagata, Masahito, Tokyo (JP); Tanaka, Toru, Tokyo (JP)
(74) Representative: Albrecht, Thomas, Dr.

(57) **Abstract**

An agglutination assay method for quantitatively determination of an analyte in an aqueous liquid sample using particles bearing an anti-analyte. The agglutination is conducted in the porous medium layer of the analysis element. A speedy quantitative analysis of the analyte can be conveniently attained with high sensitivity. When the particle-labeled anti-analyte is contained in the porous medium layer, the anti-analyte can be stored with higher stability in the dry state. A dry analysis element for enabling such analysis method is also provided.

## Description

### FIELD OF THE INVENTION

The present invention relates to a method for detecting and analyzing a trace substance by utilizing the agglutination assay, in which an analyte reacts with a particle-labeled anti-analyte, such as an antibody, to cause the particle agglutination. Particularly, the present invention relates to a dry analysis method for causing the agglutination of the particles bearing the anti-analyte in a layer construction of a dry analysis element. Also, the present invention relates to a dry analysis element which enables such analysis method.

### BACKGROUND OF THE INVENTION

In recent years, it has come to be very important to quantitatively analyze a trace substance, particularly antibody or antigen, in a specimen promptly, conveniently and precisely in order to diagnose the condition of diseases or judge the effects of treatment. For this purpose, widely employed has been an immuno-serological test for assaying the existence of an antigen or antibody in the body fluid, in which the antibody or antigen is adsorbed and immobilized to insoluble carrier particles and the resulting particles are reacted with the antigen or antibody.

The latex particles agglutination immunoassay is performed routinely by mixing a suspension of antibody-coated latex particles (sensitized latex) with a specimen on a glass plate. The latex particles agglutinate, or fail to agglutinate, as a result of interacting with the analyte antigen in the specimen. The extent of the agglutination can be determined by visual inspection. This assay makes it possible to semi-quantitatively analyze the antigen in the specimen by diluting the specimen at various ratios similar to another qualitative assay.

In Japanese Patent Publication Nos. 11575/1983 (corresponding to USP 4,118,192), 43138/1987 (corresponding to DE 2749956A1) and 55013/1987 (corresponding to DE 2749956A1), there is proposed a method, in which latex particles having an antibody bound thereto is reacted with the antigen in the sample and the amount of the agglutination of the latex particles is determined optically by nephelometry. According to the proposed method, an antigen or antibody has come to be analyzed quantitatively by an automatic analyzer.

In addition, Unexamined Japanese Patent Publication (KOKAI) Nos. 141665/1990, 94719/1994 and 213891/1994 disclose a method wherein an antigenic substance is detected by measuring a change in the absorbance upon the agglutination of the colloidal gold-labeled antibodies.

The above-described immunoassays do not require B/F separation and in this point, they are useful. The latex reagent is, however, poor in storage stability, since it is in the liquid form. In the colloidal gold agglutination, the colloidal gold solution or dispersion is not suitable as a reagent because of poor storage stability. A colloidal gold-labeled reagent in the lyophilized form must be mixed with a dedicated solution upon measurement, which makes the operation cumbersome. This method is also accompanied with such a drawback as unsuitability for use in the measurement of a small amount of a sample.

A so-called dry analysis method is, on the other hand, superior in storage stability and convenient operation. The so-called wet system (or solution system) comprises dissolving a reagent to be used for the assay in an aqueous solvent, thereby preparing the corresponding reagent solution, adding this reagent solution to a sample to be analyzed and then measuring the color reaction product by a colorimeter. On the other hand, the dry analysis method comprises spotting an aqueous sample directly to a dry analysis element, such as test piece, analytical slide or analytical tape, having a reagent composition incorporated therein in the dry form and effecting colorimetry of the color development or color change occurring in the element. The dry system is superior to the wet system using a reagent solution in convenient operation and speedy assay.

A method for causing agglutination in the layer of a dry analysis element, thereby directly detecting the existence of an agglutinate itself in the layer construction has not yet been proposed.

A dry analysis method, so-called solid phase immuno-chromatography method has also been proposed (for example, Unexamined Japanese Patent Publication (KOKAI) No. 5326/1997, which corresponds to EP 0834741A1). This method utilizes a chromatographic medium which is a liquid-permeable material serving a capillary action. The liquid-permeable sheet such as filter paper has a sample feeding zone and a detection zone, the sample feeding zone containing an colloidal gold-labeled antibody, and the detection zone containing an immobilized second antibody for binding to the different epitope of the analyte antigen. The second antibody is used as a capturing antibody. When a test sample containing an analyte antigen is fed to the sample feeding zone containing the colloidal gold-labeled antibody, the analyte antigen reacts with the colloidal gold-labeled antibody to form an immunocomplex. The formed complex diffuses and migrates to the detection zone containing the immobilized second antibody, by the capillary action of the chromatographic medium. The complex of the antigen and colloidal gold-labeled antibody are captured by the immobilized second antibody. The existence of the analyte antigen is confirmed by detecting the color tone of the colloidal gold which appears in the detection zone containing the capturing second antibody. Since the reagent used in this method is maintained at dry condition just before assay, it is excellent in storage stability. However, it is a problem that the result is not quantitative. Furthermore, in principle, the method is a sandwich method in which a colloidal gold-labeled antibody is captured by a second antibody through intervening analyte antigen, it is necessary to use the permeable medium sheet having a sufficiently large area so that an excessive colloidal gold-labeled antibody is diffused and removed from the detection zone to which the capturing second antibody is immobilized. This is the reason why the method is called as immunochromatography method. Accordingly, a plenty of liquid must be fed to the sheet, and it is necessary to use a large medium sheet. In addition, the immunochromatography method requires a long assay time, since it takes enough time for removal of an excessive colloidal gold-labeled antibody from the capturing zone by the capillary action.

Under such a circumstance, the present inventors have attempted to search for a material capable of causing agglutination in a layer medium of a dry analysis element. As a result, the inventors have found that agglutination can be caused in a porous medium layer, which is used as a spreading layer of an analysis element, and measured quantitatively with good sensitivity. By incorporating a labeling antibody into the porous medium layer, storage stability of the reagent, which is an important characteristic of a dry analysis element, is also successfully attained.

### SUMMARY OF THE INVENTION

The present invention has been accomplished in view of the aforementioned circumstances, and a first object thereof is to provide a dry analysis method for determining an analyte using an agglutination of the particles bearing an anti-analyte, by which a high sensitive analysis is ensured while using a simple operation and reagent can be stored with excellent stability in the dry state.

A second object of the present invention is to provide a dry analysis element which can detect agglutination caused by the reaction between an analyte and an anti-analyte labeled with a labeling particle, thereby analyzing the analyte in a convenient and highly sensitive manner.

The first object of the present invention is attained by an agglutination assay method for quantitative determination of an analyte in an aqueous liquid sample using particles bearing an anti-analyte, the anti-analyte being capable of specifically binding to the analyte so as to cause agglutination of the particles, comprising:
supplying said sample, together with said particles, to a porous medium layer to cause the agglutination of said particles in said porous medium layer; and
measuring the extent of the agglutination of the particles in the porous medium layer to determine the amount of the analyte in the sample.

In the present invention, the agglutination of particles bearing an anti-analyte (such as a colloidal metal-labeled antibody, which is also referred to as labeling particle or carrier) in voids of a porous medium layer. Since a liquid sample can be kept in these voids of the porous medium layer, the agglutination can be caused with a large amount of the liquid sample per unit area. Therefore, the detection of the agglutination with higher sensitivity is expected. Further, by incorporating particles bearing an anti-analyte into the porous medium layer in advance, the porous medium layer can be made dry state to an extent not harmful to stability thereof upon storage. Upon analysis, the porous medium layer is wetted with an aqueous test sample and thereby provides a reaction field sufficient for causing agglutination of labeling particles bearing an anti-analyte.

The labeling particles may be fed, together with an analyte upon assay, to the porous medium layer. Alternatively, the particles bearing an anti-analyte may be incorporated in the porous medium layer in advance, and the particles bearing an anti-analyte may be subjected to agglutination by the immunoreaction with an analyte.

The agglutination caused in the porous layer is detected as an optical change of the transmitted or reflected light. The existence of the agglutinate and its amount may be detected as a turbidity change in the porous layer medium, or as a change in color tone of the labeling particle due to agglutination. The porous layer preferably has an optical characteristics which allows the detection of turbidity change, coloration, or change in color tone from outside of the layer.

The second object of the present invention is attained by a dry analysis element for quantitative determination of an analyte in an aqueous liquid sample by measuring the extent of agglutination of particles bearing an anti-analyte, the anti-analyte being capable of specifically binding to the analyte so as to cause agglutination of the particles, comprising:
a porous medium layer containing said particles bearing the anti-analyte;
whereby, when the sample is applied to the porous medium layer, the agglutination of said particles takes place in the porous medium layer.

Preferably, the porous medium layer may serve as a so-called spreading layer. The porous medium layer may be formed of a fibrous or non-fibrous material which is used for a spreading layer.

### BRIEF DESCRIPTION OF THE DRAWINGS

Fig. 1 is an illustration showing the layer structure of one embodiment of the dry analysis element according to the present invention; and
Fig. 2 is a graphic representation showing the results of Example 1, more specifically, the calibration curve of dry analysis elements of the slide 1 obtained in the Example 1.

### DETAILED DESCRIPTION OF THE INVENTION

### Analyte and Anti-Analyte

As an analyte or a substance to be analyzed in the present invention, any substance can be used insofar as there exists a specific binding partner or substance thereto in the nature or such a substance can be prepared by chemical means. The anti-analyte, i.e., specific binding partner or substance as used herein means a substance which can specifically recognize and bind to the analyte and at the same time, can be bound to a labeling carrier particle.

Examples of the combination of an analyte and an anti-analyte thereto include combinations of antigen and antibody, a certain saccharide and lectin, biotin and avidin, protein A and IgG, hormone and receptor thereof, enzyme and substrate, and nucleic acid and complementary nucleic acid. In the above-exemplified combinations, the analyte and the anti-analyte may be reversed.

The most ordinary example is a combination of an antigen as an analyte and an antibody as an anti-analyte. The antibody as an anti-analyte may be either a polyclonal or monoclonal antibody. Alternatively, a plurality of different antibodies can be used. No particular limitation is imposed on the class of the antibody and it does not matter whether it is IgG or IgM. It may be a fragment of an antibody, for example, Fab, Fab' or F(ab')₂. When a monoclonal antibody is employed as a specific binding substance, an analyte antigen must have at least two same epitopes in order to cause agglutination of a labeling particle having an antibody bound thereon. Alternatively, at least two different antibodies which bind to different epitopes of the analyte antigen, respectively, may be bound to the labeling carrier particle. When the analyte antigen is composed of plural sub-units, such as hemoglobin, however, there is no need to use plural different monoclonal antibodies. Binding plural molecules of single kind of monoclonal antibody to the labeling carrier (particle), the agglutination of the particles can be caused by the reaction with the analyte antigen. At least two antibody molecules are preferably bound to the labeling carrier (particle) for causing agglutination.

### Labeling Particle

As a labeling carrier or particle for labeling by binding an anti-analyte, any particle can be used insofar as it undergoes agglutination as a result of reaction with the analyte and the anti-analyte bound to the particle and the extent of the agglutination falls within a detectable range. As the labeling particle, those ordinarily employed for immuno-agglutination can be used. Examples of the carrier particle include organic high-molecular latex particles such as polystyrene or styrene-butadiene copolymer, and metals such as colloidal metal. The labeling particles (or colloid) are preferred to have an average particle size falling within a range of 0.02 to 10 µm. When the particles have an excessively large particle size, optical strength due to optical reflection or light scattering of the particle itself prior to the immunoreaction becomes too high, resulting in difficulty in measurement of the change of the optical density. Too small particle sizes, on the other hand, tend to lower the detection sensitivity of the agglutinate.

Any conventionally known colloidal metal can be used as a labeling particle. Examples include colloidal gold, colloidal silver, colloidal platinum, colloidal iron and colloidal aluminum hydroxide. In particular, colloidal gold and colloidal silver are preferred because they color red and yellow, respectively, at a proper particle size. The particle size of a colloidal metal is preferably about 1 to 500 nm. The size of 5 to 100 nm is particularly preferred, because it permits development of a strong color tone.

The colloidal metal and the anti-analyte can be bound in a conventionally known manner (for example, The Journal of Histochemistry and Cytochemistry, Vol.30, No.7, pp 691-696 (1982)). For example, a colloidal metal and an anti-analyte (e.g., an antibody) are mixed in a proper buffer solution and incubated at room temperature for at least 5 minutes. The reaction mixture is centrifuged to remove a supernatant. The obtained precipitate is dispersed into a solution containing a dispersant such as polyethylene glycol to obtain an aimed colloidal metal bearing an anti-analyte.

In the case of using a colloidal gold particle as the colloidal metal, commercially available one may be employed. Alternatively, a colloidal gold particle can be prepared according to a conventional method, for example, a method of reducing chloroauric acid with sodium citrate (Nature Phys. Sci., Vol.241, 20(1973) etc.).

### Layer Structure of Dry Analysis Element

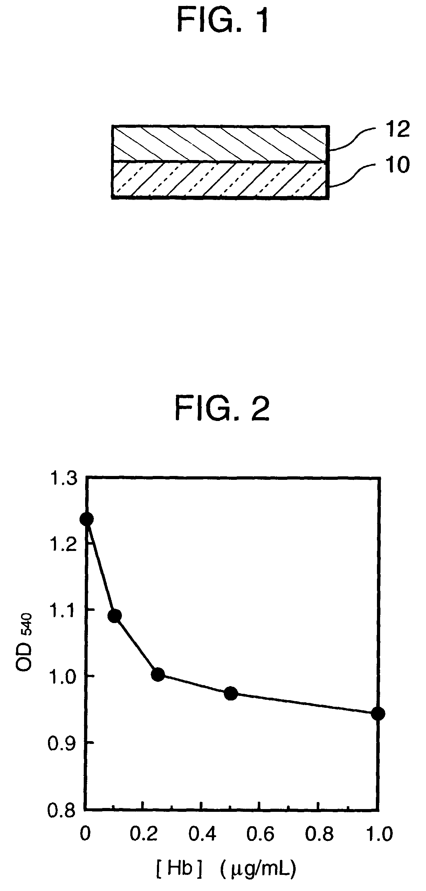

Fig. 1 shows the layer structure of one embodiment of the dry analysis element according to the invention. In Fig. 1, reference numeral 10 designates a support on which a porous medium layer 12 is laminated. The porous medium layer is also referred to as porous layer, hereinafter.

The support 10 may be light non-transmitting (opaque), light-semi-transmitting (translucent), or light-transmitting (transparent), and it is generally preferable that the support is light-transmitting and water-impermeable. Preferable materials for the light-transmitting and water-impermeable support are polyethylene terephthalate and polystyrene. In general, an undercoating is provided or the support is subjected to hydrophilization treatment in order to firmly adhere the porous medium layer 12 to be laminated thereon.

The porous medium layer 12 may be fibrous or non-fibrous. As the fibrous material, filter paper, non-woven cloth, woven cloth (e.g., plain woven cloth such as broad and poplin), knitted cloth (e.g., knitted cloth such as tricot, double tricot, and milaneaze) or filter paper made of glass fibers may be used. Examples of the non-fibrous material may be either one of a membrane filter composed of cellulose acetate as described in Unexamined Japanese Patent Publication (KOKAI) No. 53888/1974 (corresponding to USP 3,992,258), or a particulate structure layer containing interconnected voids and composed of inorganic or organic fine particles as disclosed in Unexamined Japanese Patent Publication (KOKAI) Nos. 53888/1974 (corresponding to USP 3,992,258), 90859/1980 (corresponding to USP 4,258,001) and 70163/1983 (corresponding to USP 4,486,537). A laminated structure made of partially bonded multiple porous layers may also be preferably used, examples of such structure being disclosed in Unexamined Japanese Patent Publication (KOKAI) Nos. 4959/1986 (corresponding to EP 0166365A), 116258/1987, 138756/1987 (corresponding to EP 0226465A), 138757/1987 (corresponding to EP 0226465A) and 138758/1987 (corresponding to EP 0226465A).

The porous medium layer 12 may be a spreading layer having a so-called metering function to spread, in the layer, a liquid over an area substantially in proportion to the volume of the liquid fed thereto. By providing the porous layer 12 with the function of a spreading layer, the volume of the liquid fed to and spread in the porous layer per area is made uniform and thereby accuracy at quantitative determination of the analyte by agglutination in the porous layer is improved.

Preferable materials for the spreading layer are woven and knitted fabrics. The woven fabrics or like may be subjected to the glow discharge treatment as described in Unexamined Japanese Patent Publication (KOKAI) No. 66359/1982 (corresponding to USP 4,783,315 and GB 2,087,974A). In order to adjust the area or rate for spreading, the spreading layer may contain a hydrophilic polymer or a surfactant as described in Unexamined Japanese Patent Publication (KOKAI) Nos. 222770/1985 (corresponding to EP 0162301A), 219397/1988 (corresponding to DE 3717913A), 112999/1988 (corresponding to DE 3717913A) and 182652/1987 (corresponding to DE 3717913A).

In the porous medium layer 12, a buffer may be incorporated so that the specific binding reaction between the particle-labeled anti-analyte and the analyte occurs at an optimum pH. When the binding reaction is an antigen-antibody reaction, for example, pH buffers usable for an ordinary antigen-antibody reaction can be employed. Specific examples of such buffers include buffer reagents containing tris(hydroxymethyl)aminomethane (Tris), buffer reagents containing phosphate, buffer reagents containing borate, buffer reagents containing citric acid or citrate, buffer reagents containing glycine, buffer reagents containing Bicine, buffer reagents containing HEPES, and buffer reagents containing Good's buffer agent such as MES (2-morpholinoethanesulfonic acid). The reaction may be effected at any pH insofar as the pH is within a range permitting an ordinary antigen-antibody reaction.

Further, in the porous layer 12, a high molecular polymer such as polyvinyl alcohol, polyvinyl pyrrolidone or PEG (polyethylene glycol) may be incorporated for the purpose of promoting agglutination.

The porous layer 12 may contain particles bearing an anti-analyte in advance. In this case, agglutination can be caused in the porous layer 12 by simply applying a liquid sample containing an analyte to the porous layer 12.

In addition, the porous layer 12 may contain light reflecting fine particles of, for example, titanium dioxide or barium sulfate so as to serve a light reflecting function. The porous layer 12 having the light reflecting or light shielding function may act as a white background so that change of color or color density caused by agglutination in the porous layer 12 is reflectively measured from the light-transmitting support 10 side. When the porous layer 12 itself possesses an optical property suitable for white background, the porous layer may not contain light-reflecting fine particles.

The porous layer 12 may be laminated on the support 10 via an undercoating layer (such as gelatin) formed on the support 10. At this point, partial adhesion or spot-bonding may be applied to the lamination. The terminology "partial adhesion" as used herein is a definition of the adhesion feature or mode between adjacent porous layers or between a porous layer and an adjacent non-porous layer, and means "an adhesion at the interface between adjacent two layers which are bonded substantially intimately to form an integral structure by adhesion spots arranged partially or discontinuously on the interface so as not to hinder substantially uniform passage of a liquid through and between the adjacent two layers" as in Unexamined Japanese Patent Publication (KOKAI) Nos. 4959/1986 (corresponding to EP 0166365A) and 138756/1987 (corresponding to EP 0226465A).

In general, an adhesive is spotted partially on the support, and the porous layer is placed thereon to be adhered by uniformly pressing with a light pressure. An adhesive may be spotted or partially applied on the support by any of processes as disclosed in Unexamined Japanese Patent Publication (KOKAI) Nos. 4959/1986 (corresponding to EP 0166365A), 138756/1987 (corresponding to EP 0226465A) and 23160/1989 (corresponding to DE 3721236A). A printing process is preferred than other processes. An example of the printing process is described in "INSATSU KOGAKU BINRAN" ("Handbook of Printing Technology"), edited by Japan Printing Institution and published by GIHO-DO SYUPPAN K.K. in 1983, pp 838-858. A silk screen may be employed as in the following Examples.

Known adhesives disclosed in Unexamined Japanese Patent Publication (KOKAI) No. 138756/1987 (corresponding to EP 0226465A) and on pages 839 to 853 of the aforementioned "INSATSU KOGAKU BINRAN" may be used. Examples of adhesive which may be used include water-soluble adhesives, adhesives soluble in organic solvents and thermally thermoplastic or thermosetting adhesives (hot-melt type or heat sensitive adhesives). Specific examples of the water-soluble adhesive are aqueous adhesives such as starch; aqueous solutions of dextrin, carboxymethyl cellulose and polyvinyl alcohols; and emulsion of copolymer of vinyl acetate and butyl acrylate.

In the analysis element of the present invention, partially bonding the porous layer 12 and the support 10 together does not ensure the uniform passage of a liquid between the layers. However, between the support and the porous layer, there exist interconnected minute spaces which are not filled with an adhesive. It is assumed that such spaces exhibit a sort of weak water absorbing effect. When an aqueous liquid sample is spotted on the porous layer, a liquid capable of permeating through and diffusing into the porous layer can sufficiently reach the interface between the support and the porous layer. Along with the diffusion and permeation of the liquid, an agglutinate of labeling particles formed in the porous layer can migrate to the interface efficiently. Therefore, by choosing the interface between the support and the porous layer as a measuring object, an optical change caused by agglutination can be measured with good sensitivity.

### Preparation of Dry Analysis Element

The dry analysis element of the invention may be prepared by any of the known processes described in the specifications of the aforequoted patents. The analysis element of the invention may be cut into a square piece having sides each ranging from about 15 to 30 mm or a disk having a substantially same area. It is preferred, in view of the preparation, packaging, shipping, storage and measuring operations, that the element be contained in a slide frame as descried, for example, in Japanese Patent Publication No. 28331/1982 (corresponding to USP 4,169,751), Unexamined Japanese Utility Model Publication No. 142454/1981 (corresponding to USP 4,387,990), Unexamined Japanese Patent Publication No. 63452/1982, Unexamined Japanese Utility Model publication No. 32350/1983 and Unexamined Japanese Patent publication No. 501144/1983 (corresponding to International Publication WO 83/00391) for use as a slide for chemical analysis. For the convenience in some uses, it may be formed in a long tape shape which is contained in a cassette or magazine, or a small piece thereof may be applied on or contained in a card having an opening.

### Analysis Method Using the Dry Analysis Element

The analysis element of the invention may be used for the quantitative analysis of an analyte in a sample liquid by using it through the operations described in the specifications of the aforequoted patents. When the analyte is an antigen or an antibody, about 5 µL to about 30 µL, preferably 8 µL to 15 µL, of an aqueous sample liquid such as plasma, serum or urine is spotted on the porous medium layer 12. The analysis element thus spotted is then incubated at a constant temperature of from about 20°C to about 45°C, preferably at a constant temperature of from about 30°C to about 40°C, for 1 to 10 minutes. The reflection optical density of the color or the change in color in the element may be measured from the light-transmitting support side, and the quantity of the analyte contained in the sample can be determined using a preliminarily prepared calibration curve based on the principle of colorimetry. The volume of the spotted liquid sample and the time and temperature for incubation are maintained constant to improve the accuracy in quantitative analysis.

The measuring operation may be carried out while using the chemical analysis apparatuses described in Unexamined Japanese Patent Publication Nos. 125543/1985, 220862/1985, 294367/1986 and 161867/1983 (corresponding to USP 4,424,191) to realize quantitative analysis at a high accuracy by extremely easy operation. Depending on the purpose or required precision, however, semi-quantitative measurement may be conducted by visually judging the degree of coloring or change of color tone.

When the analysis element does not contain the labeling particles bearing an anti-analyte, a necessary immunological reaction can be carried out in a proper reaction mixture other than the element, and then the resultant reaction mixture is spotted on the element. Thus the analyte can be analyzed. For assaying an antigen, for example, an aqueous sample liquid is mixed with a solution containing an antibody labeled with the labeling particle to complete the binding reaction, and then spotted on the element.

For example, when an antigen, an antibody and a colloidal metal are used as an analyte, an anti-analyte and a labeling particle, respectively, a dry analysis element can be prepared and a dry analysis using the element can be carried out as described below.

Specifically, an antibody labeled with a colloidal metal is dispersed in a buffer solution containing a proper dispersant or stabilizer. The resulting dispersion is applied to a porous medium layer, which is a spreading layer medium, and dried. The porous layer is then laminated on a light-transmitting support 10 to prepare a dry analysis element for agglutination. Alternatively, after the lamination of the porous spreading layer on the light-transmitting support, a solution containing an antibody labeled with a colloidal metal is applied to the spreading layer and dried to prepare a dry analysis element for agglutination.

An aqueous liquid sample containing an analyte (e.g., antigen) is spotted and applied onto the resulting analysis element. The analyte causes the antigen-antibody binding reaction with the colloidal metal-labeled antibody in the porous layer 12, resulting in agglutination of the colloidal metal.

Agglutination changes the color tone or hue of the colloidal metal so that the analyte in the sample can be detected and quantitatively analyzed by measuring a change in the color tone of the porous layer 12. For example, a colloidal gold before agglutination colors reddish violet having a main absorption wavelength at about 540 nm. By the agglutination, the colloidal gold increases in size, leading to shifting of its absorbance to the side of a longer wavelength, and as a result, the agglutinated colloidal gold colors pale reddish purple or gray. Accordingly, the analyte (antigen) can be quantitatively analyzed from a decrease in the reflection optical density at 540 nm, an increase in the reflection optical density at about 650 nm which appears by agglutination, or a difference between reflection optical densities at 540 nm and 630 nm.

### Example 1

### Preparation and Evaluation of Dry Analysis Element

On a colorless transparent smooth and flat polyethylene terephthalate (PET) film (support, thickness: 180 µm) undercoated with gelatin was placed a silk screen (pore size: 200 µm, space between centers of pores: 700 µm), to which an adhesive for office job (starch paste) was then applied by means of the squeeze method, followed by peeling oft the screen to form mesh points of the adhesive on the support. Then, thereon was placed a white broad woven cloth made of a polyester which had been previously immersed in 10 mM phosphate buffer (pH 7.2; supplemented with 1.0% bovine serum albumin) at room temperature for 24 hours and dried. The cloth was pressed and adhered by applying slight pressure to form a spreading layer.

An aqueous solution of the following composition was coated on the spreading layer and dried to form a reagent layer. The respective components had the coverage as set forth below.

| | |
|---|---|
| 50 mM sodium phosphate buffer (pH 7.0) | 242.3 g/m² |
| colloidal gold-labeled anti-human hemoglobin antibody | 200 mg/m² |

As the colloidal gold-labeled anti-human hemoglobin antibody, there was used a colloidal gold antibody reagent (conjugate of colloidal gold and mouse monoclonal anti-human hemoglobin antibody) of an immunological kit for detecting fecal occult hemoglobin "Immuno-Gold Hem" (produced by Godo Shusei Co., Ltd., sold by Wako Pure Chemicals Industries Ltd.).

Then, the thus prepared analysis element was cut into rectangular chips of 12 x 13 mm size. The chips were encased with slide frames described in Unexamined Japanese Patent Publication No. 63452/1982 to prepare a dry slide 1 for analysis of hemoglobin according to the present example.

A human hemoglobin A₀ (Hb) (product of Exocell. INC) was diluted with 0.2 M ammonium chloride aqueous solution (pH 6.8) containing 6% polyethylene glycol 6000 to prepare a series of diluted solutions of 0, 100, 250, 500 and 1000 ng/mL. The series of diluted solutions was spotted onto the dry slide 1 in an amount of 20 µL each. After each slide was incubated at 37°C for 5 minutes, the reflection optical density at central wavelength of 540 nm was measured from PET support side. Fig. 2 shows the calibration curve obtained.

As apparent from Fig. 2, the slide 1 of the present example exhibited that hemoglobin could be quantitatively determined at a high accuracy. Especially, the decrease of OD₅₄₀ was drastic at a low Hb concentration range. This fact shows that the slide 1 of the present example is suitable for quantitative determination of the analyte in lower concentration range and allows more highly sensitive analysis. In addition, it is confirmed that the slide 1 of the present example allows a liquid sample to be kept in the spreading layer (porous layer) upon spotting the liquid sample and thus operability is superior owing to no disturbance of a liquid flow at handling, at transportation to a measuring equipment, or at slide transportation within a measuring equipment.

### Example 2

### Storage Stability of Dry Analysis Element

The storage stability of the dry analysis element (slide 1) obtained in Example 1 was examined. A dry analysis element is generally stable at 4°C for a duration of about 1 year. In this experiment, the elements were stored in a dry incubator set up at 35°C for 0, 1, 4, 7 days after preparation of the slides as an acceleration test.

As a comparative example, 250 µg/mL of colloidal gold-labeled anti-human hemoglobin antibody solution (50 mM sodium phosphate, pH 7.0) was prepared and used as a reagent for solution-type agglutination in the comparative example. The solution reagent of the comparative example was stored in an incubator of 35°C for 0, 1, 4, 7 days after the preparation in a similar manner of the slide 1.

100 ng/mL or 500 ng/mL of a human hemoglobin solution (human hemoglobin A₀ (Hb) (product of Exocell. INC), 6% polyethylene glycol 6000, 0.2 M ammonium chloride (pH 6.8)) was spotted, in an amount of 20 µL, onto each slide 1 after storing for the prescribed days. After each slide was incubated at 37°C for 5 minutes, the reflection optical density at 540 nm was measured from the support side. From the reflection optical density obtained, hemoglobin concentration was calculated based on the calibration curve made in Example 1 at the day when the slide 1 had been prepared.

As for the comparative example, 100 µL of the solution reagent stored in the prescribed days was mixed with 50 µL of 100 or 500 ng/mL of the human hemoglobin solution. After the reaction for 10 minutes, the transmission optical density at 540 nm was measured and hemoglobin concentration was calculated based on the calibration curve made by using standard solutions in advance.

As shown in Table 1, in the case of the solution reagent (the comparative example) to be employed for conventional solution-type colloidal gold agglutination, error of the calculated Hb concentration became larger during storage at 35°C with passage of days. The error reached to about 20% at the fourth day and about 40% to 70% at the seventh day from the beginning of the storage. On the other hand, the error was about 5% to 10% at the fourth day and only 5% to 16% even at the seventh day from the beginning of the storage in the case of the slide 1. As shown in the above, the dry analysis element according to the present invention is excellent in storage stability.

**Table 1**

| Comparison of Storage Stability | | | |
|---|---|---|---|
| Hb conc. (ng/mL) | Storage time (day) | Calculated Hb conc. (ng/mL) | |
| | | Slide 1 | Comparative Example |
| 100 | 0 | 100 | 100 |
| | 1 | 95 | 105 |
| | 4 | 105 | 120 |
| | 7 | 105 | 140 |
| 500 | 0 | 500 | 500 |
| | 1 | 480 | 550 |
| | 4 | 550 | 600 |
| | 7 | 580 | 850 |

As described above, by the analysis method of the present invention, the agglutination of an analyte (e.g., antigen) with particles bearing an anti-analyte (e.g., colloidal gold-labeled antibody) can be caused in the porous medium layer of the dry analysis element. Since the agglutination is caused in the porous medium layer capable of keeping a large amount of a liquid sample per unit area, a speedy quantitative analysis of the analyte by the particle agglutination can be conveniently attained with high sensitivity.

In addition, the dry analysis element of the present invention can be a medium of dry state, upon storage, to an extent not harmful to stability of the reagent composition to be used. Upon analysis, the porous medium layer is wetted by an aqueous liquid sample and thereby can provide a reaction field to sufficiently cause agglutination of particles bearing an anti-analyte.

Furthermore, when particles bearing an anti-analyte are incorporated in the porous layer in advance, a highly sensitive dry analysis of the analyte can be conducted by simply spotting and feeding a liquid sample containing the analyte. As compared with the wet process agglutination using a conventional reagent solution, an extremely speedy and convenient analysis can be realized since preparation of the reagent at use and mixing operation are not necessary. In addition, since a liquid sample is received in the porous layer, excellent operability is ensured owing to no disturbance of a liquid flow at transportation in analysis operation.

## Claims

1. An agglutination assay method for quantitative determination of an analyte in an aqueous liquid sample using particles bearing an anti-analyte, the anti-analyte being capable of specifically binding to the analyte so as to cause agglutination of the particles, comprising:
supplying said sample, together with said particles, to a porous medium layer to cause the agglutination of said particles in said porous medium layer; and
measuring the extent of the agglutination of the particles in the porous medium layer to determine the amount of the analyte in the sample.

2. The method according to claim 1, wherein said particle is a colloidal metal and the extent of the agglutination of the particles is detected from a change in color tone of the colloidal metal caused by the agglutination.

3. The method according to claim 2, wherein said colloidal metal is colloidal gold or colloidal silver.

4. The method according to claim 1, wherein said analyte is an antigen and said anti-analyte is an antibody.

5. An agglutination assay method for quantitative determination of an analyte in an aqueous liquid sample using particles bearing an anti-analyte, the anti-analyte being capable of specifically binding to the analyte so as to cause agglutination of the particles, comprising:
providing a porous medium layer containing said particles;
supplying said sample to the porous medium layer to cause the agglutination of said particles in the porous medium layer; and
measuring the extent of the agglutination of the particles in the porous medium layer to determine the amount of the analyte in the sample.

6. The method according to claim 5, wherein said particle is a colloidal metal and the extent of the agglutination of the particles is detected from a change in color tone of the colloidal metal caused by the agglutination.

7. The method according to claim 6, wherein said colloidal metal is colloidal gold or colloidal silver.

8. The method according to claim 5, wherein said analyte is an antigen and said anti-analyte is an antibody.

9. A dry analysis element for quantitative determination of an analyte in an aqueous liquid sample by measuring the extent of agglutination of particles bearing an anti-analyte, the anti-analyte being capable of specifically binding to the analyte so as to cause agglutination of the particles, comprising:
a porous medium layer containing said particles bearing the anti-analyte;
whereby, when the sample is applied to the porous medium layer, the agglutination of said particles takes place in the porous medium layer.

10. The dry analysis element according to claim 9, wherein the porous medium layer has an optical characteristic so that a change in color tone caused by the agglutination can be detected from outside of the porous medium layer.

11. The dry analysis element according to claim 9, wherein the porous medium layer is formed of a fibrous material.

12. The dry analysis element according to claim 11, wherein the porous medium layer is formed of knitted cloth or woven cloth.

13. The dry analysis element according to claim 9, wherein the porous medium layer is formed of a non-fibrous material.

14. The dry analysis element according to claim 9, wherein the porous medium layer also serves as a spreading layer.

15. The dry analysis element according to claim 9, which further comprises a support on which the porous medium layer is laminated by means of partial adhesion.

16. The dry analysis element according to claim 9, wherein said particle is a colloidal metal and the extent of the agglutination of the particles is detected from a change in color tone of the colloidal metals caused by the agglutination.

17. The dry analysis element according to claim 16, wherein said colloidal metal is colloidal gold or colloidal silver.

18. The dry analysis element according to claim 9, wherein said analyte is an antigen and said anti-analyte is an antibody.
